# EUROPÄISCHE PATENTANMELDUNG

(11) **EP 1 302 192 A2**
(43) Veröffentlichungstag der Anmeldung: **16.04.2003**
(21) Anmeldenummer: 02022847.4
(22) Anmeldetag: 14.10.2002
(51) Int. Cl.: A61K 7/06, A61K 7/50

(54) **Kosmetische Mittel enthaltende Polyethylenglykole**

(30) Priorität: 16.10.2001 DE 10151046
(71) Anmelder: Clariant GmbH, 65929 Frankfurt am Main (DE)
(72) Erfinder: Klug, Peter, Dr., 63762 Grossostheim (DE); Henning, Torsten, Dr., 65812 Bad Soden (DE)
(74) Vertreter: Brundin, Eike, Dr.

(57) **Zusammenfassung**

Die Erfindung betrifft kosmetische Mittel enthaltend Polyethylenglykole der Formel H(OCH₂CH₂)ₙOH, worin n für eine ganze Zahl zwischen 150 und 900 steht.

Durch den Einsatz der Polyethylenglykole werden das Haut- und das Haargefühl, die Kämmbarkeit der Haare, sowie die Schaumbildung verbessert.

## Beschreibung

Die Erfindung betrifft kosmetische Mittel enthaltend Polyethylenglykole. Durch den Einsatz der Polyethylenglykole werden das Haut- und das Haargefühl, die Schaumbildung, sowie die Kämmbarkeit der Haare verbessert.

Körperreinigung und Körperpflege in zwei Schritten ist zeitraubend. Daher werden von vielen Verbrauchern Mittel mit gleichzeitig reinigender und pflegender Wirkung bevorzugt. Eine Vielzahl von kosmetischen Produkten versucht diesem Anspruch gerecht zu werden.

In US 5,612,307 werden wässrige, flüssige Körperreinigungsmittel beschrieben, die neben den üblichen Tensiden eine pflegende Komponente aus der Gruppe der Siliconöle, Fette, Öle, Wachse, hydrophoben Pflanzenextrakte, Fettsäuren, Alkohole, Ester, Lipide und Phospholipide enthalten.
In WO 94/03152 werden Duschgels beschrieben, bestehend im wesentlichen aus einem Tensid, Siliconöl und einem kationischen Polymer.
Bis dato ist jedoch unbefriedigend, dass pflegende und feuchtigkeitsspendende Komponenten nicht gleichzeitig in ausreichender Menge in wässrige kosmetische Reinigungsmittel eingearbeitet werden können.
Ein weiteres Problem besteht darin, dass sich wässrige Dispersionen aus Tensidsystemen und feuchtigkeitsspendenden und pflegenden Komponenten im Laufe der Zeit separieren und somit wenig lagerstabil sind.

J. P. Pavlichko et al. beschreiben in HAPPI, Vol 38, April 2001, S. 94-99, dass hochmolekulare Polyethylenoxide der Formel H(OCH₂CH₂)ₙOH mit n = 2 000 bis 100 000, d.h. Molmassen von ca. 88 000 bis 4 400 000 g/mol, positive Effekte in Haut- und Haarpflegeprodukten zeigen. Der Zusatz der hochmolekularen Polyethylenoxide bewirkten ein verbessertes Haut- und Haargefühl, eine Verbesserung der Schaumbildung und eine verbesserte Kämmbarkeit der Haare.

Nachteilig an den hochmolekularen Polyethylenoxiden ist jedoch, dass sie schwierig einzuarbeiten sind, da sie sehr schnell anquellen und Gele bilden.

Überraschend wurde nun gefunden, dass niedermolekulare Polyethylenglykole der Formel H(OCH₂CH₂)ₙOH mit n gleich 150 bis 900, in kosmetischen Mitteln ebenfalls ein verbessertes Haut- und Haargefühl, eine verbesserte Kämmbarkeit der Haare und eine verbesserte Schaumbildung bewirken. Dabei wirken die Polyethylenglykole als Schaumregulatoren über deren Zusatz die Cremigkeit, die Abwaschbarkeit und die Blasengröße des Schaums gesteuert werden kann. Bei der Anwendung der niedermolekularen Polyethylenglykole ist von großem Vorteil, dass sie im Gegensatz zu den in HAPPI, Vol 38, April 2001, S. 94-99 beschriebenen hochmolekularen Polyethylenoxiden einfach zu verarbeiten sind.

Gegenstand der Erfindung sind daher kosmetische Mittel, enthaltend Polyethylenglykole der Formel H(OCH₂CH₂)ₙOH, worin n für eine ganze Zahl zwischen 150 und 900 steht.

Bevorzugt sind Polyethylenglykole der Formel H(OCH₂CH₂)ₙOH, worin n für eine ganze Zahl zwischen 180 und 800, besonders bevorzugt zwischen 240 und 800, insbesondere bevorzugt zwischen 300 und 800, steht.

Bevorzugt enthalten die kosmetischen Mittel, bezogen auf die fertigen Mittel, 0,1 bis 50 Gew.-%, bevorzugt 0,5 bis 10 Gew.-%, insbesondere bevorzugt 1 bis 4 Gew.-%, der Polyethylenglykole.

In einer besonderen Ausführungsform enthalten die kosmetischen Mittel zusätzlich kationische Guar Gum Polymere, wie in der WO 97/26854 beschrieben. Überraschenderweise zeigte sich, dass durch den gleichzeitigen Einsatz der kationischen Guar Gum Polymere die Affinität der Polyethylenglykole auf der Haut signifikant gesteigert wird.
Die kationischen Guar Gum Derivate sind erhältlich durch Reaktion der Hydroxylgruppen des Polygalaktomannan-Gerüstes mit reaktiven quaternären Ammoniumverbindungen.

Bevorzugt als kationische Guar Gum Polymere sind die in der WO 97/26854 beschrieben, deren gesamter Inhalt hiermit ausdrücklich in die vorliegende Anmeldung aufgenommen wird.
Besonders bevorzugt als kationische Guar Gum Polymere sind Guar Hydroxypropyltrimethylammoniumchloride.
Bevorzugt beträgt das Molekulargewicht der kationischen Guar Gum Polymere 2 000 bis 3 000 000 g/mol.
Die fertigen kosmetischen Mittel enthalten bevorzugt 0,01 bis 1,0 Gew.-%, besonders bevorzugt 0,02 bis 0,4 Gew.-%, an kationischen Guar Gum Polymeren.

Bei den erfindungsgemäßen kosmetischen Mitteln handelt es sich bevorzugt um Shampoos, bevorzugt Haarshampoos, Spülungen, Conditioner, Cremespülungen, Cremes, Salben, Gele, Duschgels, Duschbäder und Schaumbäder.

Ein besonderer Vorteil der Polyethylenglykole hinsichtlich der Formulierungsmöglichkeiten besteht darin, dass sie sich problemlos mit allen üblichen anionischen, kationischen, zwitterionischen, nicht-ionischen und amphoteren Tensiden in wässrigen oder wässrig-alkoholischen Medien kombinieren lassen.
Dabei beträgt die Gesamtmenge an Tensiden, bezogen auf die fertigen kosmetischen Mittel, bevorzugt zwischen 5 und 70 Gew.-%, besonders bevorzugt zwischen 10 und 40 Gew.-%, insbesondere bevorzugt zwischen 12 und 35 Gew.-%.

Als anionische Tenside sind bevorzugt (C₁₀-C₂₀)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfate, Alkansulfonate und Hydroxyalkansulfonate, Olefinsulfonate, Acylester von Isethionaten,
α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Amphoacetate- oder -glycinate und/oder Acylglutamate. Die anionischen Tenside werden bevorzugt in Form ihrer wasserlöslichen oder in Wasser dispergierbaren Salze eingesetzt, z.B. als Natrium-, Kalium-, Magnesium-, Ammonium-, Mono-, Di- und Triethanolammonium- oder Alkylammonium-Salze.
Der Gewichtsanteil der anionischen Tenside beträgt, bezogen auf die fertigen erfindungsgemäßen Mittel, bevorzugt 0,1 bis 50 Gew.-%, besonders bevorzugt 7 bis 30 Gew.-%, insbesondere bevorzugt 9 bis 18 Gew.-%.

Als kationische Tenside bevorzugt sind quartäre Ammonium-Salze, bevorzugt Di-(C₁₀-C₂₄)Alkyl-dimethyl-ammonium-chlorid und -bromid, besonders bevorzugt Di-(C₁₂-C₁₈)Alkyl-dimethyl-ammonium-chlorid und -bromid; (C₁₀-C₂₄)Alkyl-dimethylethylammonium-chlorid und -bromid; (C₁₀-C₂₄)Alkyl-trimethyl-ammonium-chlorid und -bromid, bevorzugt Cetyl-trimethyl-ammonium-chlorid und -bromid und (C₂₀-C₂₂)Alkyl-trimethyl-ammonium-chlorid und -bromid; (C₁₀-C₂₄)Alkyl-dimethylbenzyl-ammonium-chlorid und -bromid, bevorzugt (C₁₂-C₁₈)-Alkyl-dimethylbenzyl-ammonium-chlorid; N-(C₁₀-C₁₈)Alkyl-pyridiniumchlorid und -bromid, bevorzugt N-(C₁₂-C₁₆)Alkyl-pyridinium-chlorid und -bromid; N-(C₁₀-C₁₈)Alkyl-isochinolinium-chlorid, -bromid und -monoalkylsulfat; N-(C₁₂-C₁₈)-Alkylpolyoylaminoformylmethyl-pyridinium-chlorid; N-(C₁₂-C₁₈)Alkyl-N-methylmorpholinium-chlorid, -bromid und -monoalkylsulfat; N-(C₁₂-C₁₈)Alkyl-N-ethylmorpholinium-chlorid, -bromid und -monoalkylsulfat; (C₁₆-C₁₈)Alkyl-pentaoxethylammonium-chlorid; Diisobutyl-phenoxyethoxyethyldimethylbenzylammonium-chlorid; Salze des N,N-Diethylaminoethylstearylamids und -oleylamids mit Salzsäure, Essigsäure, Milchsäure, Zitronensäure und Phosphorsäure; N-Acyl-aminoethyl-N,N-diethyl-N-methyl-ammoniumchlorid, -bromid und -monoalkylsulfat; und/oder N-Acylaminoethyl-N,N-diethyl-N-benzyl-ammonium-chlorid, -bromid und -monoalkylsulfat, wobei es sich bei den Acylresten vorzugsweise um Stearoyl - oder Oleoylreste handelt.
Der Gewichtsanteil der kationischen Tenside, bezogen auf die fertigen erfindungsgemäßen Mittel, beträgt bevorzugt 1 bis 10 Gew.-%, besonders bevorzugt 2 bis 7 Gew.-%, insbesondere bevorzugt 3 bis 5 Gew.-%.

Als nichtionische Tenside bevorzugt sind Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (®Pluronics);Fettsäureamldpolyethylenglykole; N-Alkyl-, N-Alkoxypolyhydroxyfettsäureamid, bevorzugt Fettsäure-N-methylglucamide und Saccharoseester; Polyglykolether; Alkylpolyglycoside; und/oder Phosphorsäureester (Mono-, Di- und Triphosphorsäureester ethoxyliert und nicht-ethoxyliert).
Der Gewichtsanteil der nichtionischen Tenside, bezogen auf die fertigen Mittel, beträgt bevorzugt 1 bis 20 Gew.-%, besonders bevorzugt 2 bis 10 Gew.-%, insbesondere bevorzugt 3 bis 7 Gew.-%.

Als Amphotenside bevorzugt sind N-(C₁₂-C₁₈)Alkyl-β-aminopropionate und N-(C₁₂-C₁₈)Alkyl-β-iminodipropionate als Alkali- und Mono-, Di- und Trialkylammonium-Salze; N-Acylaminoalkyl-N,N-dimethyl-acetobetain, bevorzugt N-(C₈-C₁₈)Acyl-aminopropyl-N,N-dimethylacetobetain; (C₁₂-C₁₈)-Alkyl-dimethylsulfopropyl-betain; Amphotenside auf Basis Imidazolin (Handelsnamen Miranol®, Steinapon®), bevorzugt das Natrium-Salz des 1-(β-Carboxy-methyloxyethyl)-1-(carboxymethyl)-2-lauryl-imidazoliniums; Aminoxide, z.B.
(C₁₂-C₁₈)Alkyl-dimethylaminoxide; und/oder Fettsäureamidoalkyl-dimethylaminoxide. Der Gewichtsanteil der amphoteren Tenside, bezogen auf die fertigen Mittel, beträgt bevorzugt 0,5 bis 20 Gew.-%, besonders bevorzugt 1 bis 10 Gew.-%.

Des weiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine, Sulfobetaine, Aminoxide, Fettsäurealkanolamide und Polyhydroxyamide eingesetzt werden.

Besonders bevorzugte Tenside sind Laurylsulfat, Laurethsulfat, Cocoamidipropylbetain, Natriumcocoylglutamat, Di-natriumlaurethsulfosuccinat und/oder Cocosfettsäurediethanolamid.

Weiterhin können die Mittel Überfettungsmittel, Fette, Wachse, Stabilisatoren, biogene Wirkstoffe, Glycerin, Konservierungsmittel, Perlglanzmittel, Farb- und Duftstoffe, Lösungsmittel, Trübungsmittel, Verdickungsmittel, Dispergiermittel, Eiweißderivate (z.B. Gelatine), Collagenhydrolysate, Polypeptide auf natürlicher und synthetischer Basis, Eigelb, Lecithin, Lanolin, Lanolinderivate, Fettalkohole, Silicone, deodorierende Mittel, Stoffe mit keratolytischer und keratoplastischer Wirkung, Enzyme, Trägersubstanzen, feuchtigkeitsspendende Stoffe und/oder antimikrobiell wirkende Agentien enthalten.

Als Überfettungsmittel bevorzugt sind polyethoxylierte Lanolinderivate, Lecithinderivate, Polyolfettsäureester, Monoglyceride und Fettsäurealkanolamide, wobei letztere gleichzeitig als Schaumstabilisatoren geeignet sind.

Bevorzugte als Fette sind Glyceride; als Wachse kommen u.a. Bienenwachs, Paraffinwachs oder Mikrowachse, gegebenenfalls in Kombination mit hydrophilen Wachsen, z.B. Cetylstearylalkohol, in Frage.

Als Stabilisatoren können Metallsalze von Fettsäuren, wie z.B. Magnesium-, Aluminium- und/oder Zinkstearat, eingesetzt werden.

Unter biogenen Wirkstoffen sind beispielsweise Pflanzenextrakte und Vitaminkomplexe zu verstehen.
Als Konservierungsmittel eignen sich beispielsweise Phenoxyethanol, Formaldehydlösung, Parabene, Pentandiol und Sorbinsäure.

Als Farbstoffe können die für kosmetische Zwecke geeigneten und zugelassenen Substanzen verwendet werden.

Als Verdickungs- und Dispergiermittel bevorzugt sind Natrium-, Kalium-, Ammoniumchlorid, Natriumsulfat, Fettsäurealkylolamide, Cellulosederivate, beispielsweise Hydroxyethylcellulose, Guar Gum, Polyvinylalkohol, Polyvinylpyrrolidon, Hydroxypropyl guar gum, Stärke und Stärkederivate, sowie natürliche Gummen, Carboxyvinylpolymere (z.B. die Carbopol®-Typen 934, -940, -941,-956,-980,-981, -1342 und -1382).

Als Verdickungs- und Dispergiermittel besonders geeignet sind Ethylenglykolester von Fettsäuren mit 14 bis 22, besonders bevorzugt 16 bis 22 Kohlenstoffatomen, insbesondere Mono- und Di-ethylenglycolstearat. Bevorzugt sind auch Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl-(C₁₂-C₂₂)-, bevorzugt (C₁₆-C₁₈),-amidobenzoesäure und deren lösliche Salze und/oder N,N-di(C₁₆-C₁₈)-amidobenzoesäure und deren Derivate.

Die Verdickungs- und Dispergiermittel werden bevorzugt in Konzentrationen, bezogen auf die fertigen Mittel, von 0,5 bis 10 Gew.-%, besonders bevorzugt 0,5 bis 5 Gew.-%, insbesondere bevorzugt 1 bis 4 Gew.-%, eingesetzt.

Die gewünschte Viskosität der Mittel kann durch Zugabe von Wasser und/oder organischen Lösungsmitteln oder durch Zugabe einer Kombination aus organischen Lösungsmitteln und Verdickungsmitteln eingestellt werden.
Als organische Lösungsmittel kommen prinzipiell alle ein- oder mehrwertigen Alkohole und ethoxylierten Alkohole in Betracht. Bevorzugt werden Alkohole mit 1 bis 4 Kohlenstoffatomen, wie Ethanol, Propanol, Isopropanol, n-Butanol und IsoButanol, Glycerin und Mischungen aus den genannten Alkoholen.
Weitere geeignete Lösungsmittel sind beispielsweise Triacetin (Glycerintriacetat) und 1-Methoxy-2-propanol.

Die erfindungsgemäßen Mittel enthalten, bezogen auf die fertigen Mittel, die Alkohole in Mengen von 0,1 bis 50 Gew.-%.

Als Trägermaterialien eignen sich bevorzugt pflanzliche Öle, natürliche und gehärtete Öle, Wachse, Fette, Wasser, Alkohole, Polyole, Glycerol, Glyceride, flüssige Paraffine, flüssige Fettalkohole, Sterol, Cellulose und Cellulose-Derivate.

Als fungizide Wirkstoffe können Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Octopyrox eingesetzt werden.

Als deodorierende Stoffe können Allantoin und Bisabolol, bevorzugt in Gewichtsmengen von 0,0001 bis 10 Gew.-%, in Frage.

Als kationische Polymere bevorzugt sind kationische Cellulosederivate, kationische Stärke, Copolymere von Diallylammoniumsalzen und Acrylamiden, quaternierte Vinylpyrrolidon/ Vinylimidazol-Polymere, Kondensationsprodukte von Polyglykolen und Aminen, quaternierte Kollagenpolypeptide, quaternierte Weizenpolypeptide, Polyethylenimin, kationische Siliconpolymere, wie z.B. Amidomethicone, Copolymere der Adipinsäure und Dimethylaminohydroxypropyldiethylentriamin, Polyaminopolyamid und kationische Chitinderivate, wie beispielsweise Chitosan. Geeignete Siliconverbindungen sind beispielsweise Dimethylpolysiloxan, Methylphenylpolysiloxane, cyclische Silicone sowie amino-, fettsäure-, alkohol-, polyether-, epoxy-, fluor- und/oder alkylmodifizierte Siliconverbindungen, sowie Polyalkylsiloxane, Polyalkylarylsiloxane, Polyethersiloxan-Copolymere, wie in US 5,104,645 und den darin zitierten Schriften beschrieben, die bei Raumtemperatur sowohl flüssig als auch harzförmig vorliegen können.

Die erfindungsgemäßen Mittel können mit konventionellen Ceramiden, Pseudoceramiden, Fettsäure-N-alkylpolyhydroxyalkylamide, Cholesterin, Cholesterinfettsäureestern, Fettsäuren, Triglyceriden, Cerebrosiden, Phospholipiden und ähnlichen Stoffen, abgemischt werden.

Als perlglanzgebende Verbindungen geeignet sind Fettsäuremonoalkanolamide; Fettsäuredialkanolamide; Monoester und Diester von Alkylenglycol, insbesondere solche aus Ethylenglykol, Propylenglycol oder deren Oligomeren und höheren Fettsäuren, z.B. Palmitinsäure, Stearinsäure, Behensäure oder Mischungen davon; Mono- oder Diester von Alkylenglykolen mit Fettsäuren; Fettsäuren und deren Metallsalze; Monoester oder Polyester von Glycerin mit Carbonsäuren; und/oder Ketosulfone.
Als perlglanzgebende Komponente besonders bevorzugt sind Ethylenglycoldistearat und Polyethylenglykoldistearat mit 3 Glykoleinheiten.

Als feuchtigkeitsspendende Substanz bevorzugt sind Isopropylpalmitat, Glycerin und/oder Sorbitol, bevorzugt in Gewichtsmengen, bezogen auf die fertigen Mittel, von 0,1 bis 50 Gew.-%.
Ebenfalls Gegenstand der Erfindung ist die Verwendung von Polyethylenglykolen der Formel H(OCH₂CH₂)ₙOH, worin n für eine ganze Zahl zwischen 150 und 900 steht, als Schaumregulator in kosmetischen Mitteln.
Dabei ist die Wirkung als Schaumregulator so zu verstehen, dass über die Polyethylenglykole die Cremigkeit, die Stabilität, die Feinheit und die Abwaschbarkeit des Schaums der kosmetischen Mittel gesteuert werden kann. Je höher die Molmasse und die Menge des eingesetzten Polyethylenglykols ist, desto cremiger und feiner fällt der Schaum aus. Die Abwaschbarkeit ist mit niedriger Molmasse besser als mit hoher Molmasse.

Ebenfalls Gegenstand der Erfindung ist die Verwendung von Polyethylenglykolen der Formel H(OCH₂CH₂)ₙOH, worin n für eine ganze Zahl zwischen 150 und 900, in kosmetischen Mitteln zur Verbesserung des Hautgefühls und Haargefühls.

Weiterhin Gegenstand der Erfindung ist ein Verfahren zur Verbesserung der Kämmbarkeit, insbesondere der Naß-Kämmbarkeit, von Haaren, umfassend die Behandlung der Haare mit einem kosmetischen Mittel, enthaltend mindestens ein Polyethylenglykol der Formel H(OCH₂CH₂)ₙOH, worin n für eine ganze Zahl zwischen 150 und 900 steht.
Verbesserte Kämmbarkeit ist dabei so zu verstehen, dass durch die Verwendung der Polyethylenglykole der mechanische Kämmwiderstand verringert wird.
Bei den kosmetischen Mitteln handelt es sich bevorzugt Haarbehandlungsmittel, besonders bevorzugt um Shampoos und Spülungen, die entsprechend den allgemein bekannten Prozeduren angewendet werden.

Für die erfindungsgemäßen Verwendungen als Schaumregulator und zur Verbesserung des Hautgefühls, sowie das erfindungsgemäße Verfahren zur Verbesserung der Kämmbarkeit der Haare eignen sich bevorzugt Polyethylenglykole der Formel H(OCH₂CH₂)ₙOH, worin n für eine ganze Zahl zwischen 180 und 800, bevorzugt zwischen 240 und 800, besonders bevorzugt zwischen 300 und 800, steht.

Die nachfolgenden Beispiele sollen den Gegenstand der Erfindung näher erläutern, ohne ihn darauf einzuschränken.

### Beispiele

Austestung von Schaumverhalten, Hautgefühl, Haargefühl und Kämmbarkeit der Haare:

In einem doppelblind Sensorik-Paneltest (10 Testpersonen) wurden Tensid-Formulierungen Ethersulfat/Betain (Verhältnis Aktivgehalt 7:3) mit Polyglykol 35000 S (Testformulierung) und ohne Polyglykol 35000 S (Standardformulierung) geprüft. Die Ausprüfung erfolgte auf einer Skala von -1,5 (max. negative Bewertung) bis +1,5 (max. positive Bewertung).

| Rezeptur der Tensidbasis: | Standard | Testformulierung |
|---|---|---|
| Sodium Laureth Sulfate (27 % aktiv) | 48,1 Gew.-% | 48,1 Gew.-% |
| Cocamidopropyl Betaine (30 % aktiv) | 6,7 Gew.-% | 6,7 Gew.-% |
| Polyglykol 35000 S | - | 0,5 Gew.-% |
| E-Wasser | ad 100 | ad 100 |
| NaCI | 1,8 Gew.-% | 2,1 Gew.-% |

### Ausprüfung:

| 1) Schaumverhalten: | Standard | Testformulierung |
|---|---|---|
| Anschäumverhalten (schnell/langsam) | 0,4 | 0,5 |
| Verteilbarkeit (leicht/schwierig) | 0,6 | 0,8 |
| Schaummenge (hoch/niedrig) | 0,1 | 0,3 |
| Schaumblasengröße (fein/grob) | 0,4 | 0,7 |
| Schaum Cremigkeit (cremig/wässrig) | 0,2 | 0,7 |
| Abwaschbarkeit des Schaums (leicht/schwierig) | 0,5 | 0,6 |
| | | |

| 2) Hautgefühl: | Standard | Testformulierung |
|---|---|---|
| Hautgefühl nass (glatt/stumpf) | 0 | 0,5 |
| Hautgefühl feucht (glatt/stumpf) | -0,6 | 0,2 |
| Pflegeempfinden getrocknete Haut (weich/trocken) | -0,2 | 0,7 |
| Glätte der getrockneten Haut (glatt/stumpf) | -0,2 | 0,8 |
| | | |

| 3) Haargefühl: | Standard | Testformulierung |
|---|---|---|
| Haargefühl (geschmeidig/stumpf) | 0 | 0,3 |
| | | |

| 4) Kämmbarkeit der Haare: | Standard | Testformulierung |
|---|---|---|
| Kämmbarkeit trocken (gut/schlecht) | -1 | 0,3 |
| Kämmbarkeit nass (gut/schlecht) | -0,8 | 0,5 |

### Ergebnis:

Die Testformulierung mit Polyglykol 35000 S erhielt eine Gesamtpunktzahl von +6,9 und schnitt damit deutlich besser ab als die Standardformulierung mit -0,6 Punkten. Besonders signifikante Unterschiede wurden bei der Cremigkeit des Schaums, dem Hautgefühl (nass, feucht und trocken), der Hautglätte, sowie der Naß-Kämmbarkeit beobachtet.

### Formulierungsbeispiel 1: Duschgel

| Komponenten | | Menge in Gew.-% |
|---|---|---|
| 1 | Carbopol ETD 2020 | 1,5 |
| 2 | Polyquaternium-10 | 0,3 |
| 3 | Glycerin | 2,0 |
| 4 | Polyglykol 35000 S | 2,0 |
| 5 | Genagen LDA | 9,2 |
| 6 | Genagen CAB | 4,0 |
| 7 | Hostapon CLG | 4,8 |
| 8 | Zitronensäure | 0,5 |
| 9 | Methyldibromoglutaronitril/Phenoxyethanol | 0,05 |
| 10 | Parfüm | 0,5 |
| 11 | Trübungsmittel Opacifyer 641 | 0,8 |
| 12 | E-Wasser | ad 100 |

### Herstellung:

Komponente 1 und 2 wurden vorgelegt und in ca. 70°C heißem E-Wasser unter Rühren gelöst. Nacheinander wurden die Komponenten 3, 4, 5, 6 und 7 unter Rühren zugegeben und der pH-Wert mit Zitronensäure auf pH 6,0 eingestellt. Durch Zugabe der Komponenten 9 und 10 wurde das Mittel konserviert und parfümiert. Anschließend wurde das Trübungsmittel 11 zugegeben.

### Formulierungsbeispiel 2: Duschgel

| Komponenten | | Menge in Gew.-% |
|---|---|---|
| 1 | Carbopol ETD 2020 | 3,0 |
| 2 | Polyquaternium-10 | 0,3 |
| 3 | Polyglykol 20000 S | 3,0 |
| 4 | Medialan LD | 2,0 |
| 5 | Genagen LAA | 7,2 |
| 6 | Genagen CAB | 4,0 |
| 7 | Hostapon KCG | 6,9 |
| 8 | Milchsäure | 0,5 |
| 9 | Konservierungsmittel | q.s. |
| 10 | Parfüm | q.s. |
| 11 | Genapol TSM | 1,0 |
| 12 | E-Wasser | ad 100 |

### Herstellung:

Die Komponenten 1 und 2 wurden vorgelegt und in ca. 70°C heißen E-Wasser unter Rühren gelöst. Nacheinander wurden die Komponenten 3, 4, 5, 6 und 7 unter Rühren zugegeben und der pH-Wert mit Milchsäure auf pH 6,0 eingestellt. Durch Zugabe der Komponenten 9 und 10 wurde das Mittel konserviert und parfümiert. Anschließend wurde das Seidenglanzmittel 11 zugegeben.

### Formulierungsbeispiel 3: Haarshampoo

| Komponenten Menge in Gew.-% | | |
|---|---|---|
| 1 | Genapol LRO liquid | 11,10 |
| 2 | Duftstoff | 0,30 |
| 3 | E-Wasser | ad 100 |
| 4 | Genagen CAB | 24,00 |
| 5 | Genagen LAA | 11,60 |
| 6 | Polyglykol 35000 S | 2,00 |
| 7 | Zitronensäure (50 %ig in Wasser) | 1.20 |
| 8 | Farbstofflösung | q.s. |
| 9 | Konservierungsmittel | q.s. |

### Herstellung:

Die Komponente 1 wurde vorgelegt, anschließend wurden die anderen Komponenten in der angegebenen Reihenfolge eingerührt.

### Formulierungsbeispiel 4: Cremespülung

| Komponenten | | Menge in Gew.-% |
|---|---|---|
| 1 | Genamin DSAC | 1,50 |
| 2 | Hostacerin T-3 | 1,50 |
| 3 | Cetylalkohol | 2,50 |
| 4 | Paraffinöl, hochviskos | 1,00 |
| 5 | Genamin KSL | 2,00 |
| 6 | E-Wasser | 91,20 |
| 7 | Polyglykol 20000 S | 2,00 |
| 8 | Konservierungsmittel | q.s. |
| 9 | Parfümöl | 0,30 |
| 10 | Farbstofflösung | q.s. |
| 11 | Zitronensäure | q.s. |

### Herstellung:

Die Komponenten 1 bis 4 wurden bei ca. 75°C aufgeschmolzen. Anschließend wurden die Komponenten 5 bis 8 auf ca. 75°C erwärmt und unter Rühren zugesetzt. Anschließend wurde kaltgerührt. Danach wurden bei ca. 35°C die Komponenten 9 und 10 eingerührt und schließlich wurde der pH-Wert mit Zitronensäure auf pH 4 eingestellt.

### Verzeichnis der eingesetzten Produkte:

| | | |
|---|---|---|
| Carbopol®ETD 2020 | (Goodrich) | Polyacrylsäure, vernetzt |
| Polyquaternium-10 | | kation. modifizierter Celluloseether |
| Polyglykol 20000 S | (Clariant GmbH) | PEG-350, Polyethylenglykol mit mittlerer Molmasse 20000 g/mol |
| Polyglykol 35000 S | (Clariant GmbH) | PEG-800, Polyethylenglykol mit mittlerer Molmasse 35000 g/mol |
| Genagen® LDA | (Clariant GmbH) | Laurylamphodiacetat, Na-Salz |
| Genagen® LAA | (Clariant GmbH) | Laurylamphoacetat, Na-Salz |
| Genagen® CAB | (Clariant GmbH) | Cocoamidopropylbetain |
| Hostapon® CLG | (Clariant GmbH) | Natriumlaurylglutamat |
| Hostapon®KCG | (Clariant GmbH) | Natriumcocoylglutamat |
| Medialan® LD | (Clariant GmbH) | Natriumlauroylsarcosinat |
| Genapol® TSM | (Clariant GmbH) | PEG-3 Distearat, Natriumlaurethsulfat |
| Opacyfier® 641 | (Lytron) | Sodium Styrene/Acrylates Copolymer |
| Genapol® LRO | (Clariant GmbH) | Sodium Laureth Sulfate |
| Genamin® KSL | (Clariant GmbH) | PEG-5 Stearyl Ammonium Lactate |
| Hostacerin® T-3 | (Clariant GmbH) | Ceteareth-3 |
| Genamin® DSAC | (Clariant GmbH) | Distearyldimonium Chloride |

## Patentansprüche

1. Kosmetische Mittel, enthaltend Polyethylenglykole der Formel H(OCH₂CH₂)ₙOH, worin n für eine ganze Zahl zwischen 150 und 900 steht.

2. Mittel nach Anspruch 1, **dadurch gekennzeichnet, dass** n für eine ganze Zahl zwischen 180 und 800, bevorzugt zwischen 240 und 800, besonders bevorzugt zwischen 300 und 800, steht.

3. Mittel nach Anspruch 1 und/oder 2, **dadurch gekennzeichnet, dass** die fertigen Mittel die Polyethylenglykole in Mengen von 0,1 bis 50 Gew.-%, bevorzugt 0,5 bis 10 Gew.-%, insbesondere bevorzugt 1 bis 4 Gew.-%, enthalten.

4. Mittel nach mindestens einem der Ansprüche 1 bis 3, **dadurch gekennzeichnet, dass** die Mittel zusätzlich kationische Guar Gum Polymere enthalten.

5. Mittel nach Anspruch 4, **dadurch gekennzeichnet, dass** es sich bei den kationischen Guar Gum Polymeren um Guar Hydroxypropyltrimethylammonium Chloride handelt.

6. Mittel nach Anspruch 4 und/oder 5, **dadurch gekennzeichnet, dass** die Menge an kationischen Guar Gum Polymeren, bezogen auf die fertigen Mittel, 0,01 bis 1,0 Gew.-%, bevorzugt 0,02 bis 0,4 Gew.-%, beträgt.

7. Mittel nach mindestens einem der Ansprüche 1 bis 6, **dadurch gekennzeichnet, dass** es sich dabei um Shampoos, bevorzugt Haarshampoos, Spülungen, Conditioner, Cremespülungen, Cremes, Salben, Gele, Duschgels, Duschbäder und Schaumbäder handelt.

8. Verwendung von Polyethylenglykolen der Formel H(OCH₂CH₂)ₙOH, worin n für eine ganze Zahl zwischen 150 und 900 steht, als Schaumregulatoren in kosmetischen Mitteln.

9. Verwendung von Polyethylenglykolen der Formel H(OCH₂CH₂)ₙOH, worin n für eine ganze Zahl zwischen 150 und 900 steht, in kosmetischen Mitteln zur Verbesserung des Hautgefühls und Haargefühls.

10. Verfahren zur Verbesserung der Kämmbarkeit, insbesondere der Naß-Kämmbarkeit, von Haaren, umfassend die Behandlung der Haare mit einem kosmetischen Mittel, enthaltend mindestens ein Polyethylenglykol der Formel H(OCH₂CH₂)ₙOH, worin n für eine ganze Zahl zwischen 150 und 900 steht.

11. Verwendung oder Verfahren nach einem der Ansprüche 8, 9 oder 10, **dadurch gekennzeichnet, dass** n für eine ganze Zahl zwischen 180 und 800, bevorzugt zwischen 240 und 800, besonders bevorzugt zwischen 300 und 800, steht.
